Europäisches Patentamt

European Patent Office

Office européen des brevets

(11) Numéro de publication: **0 023 186**
**B1**

(12) **FASCICULE DE BREVET EUROPEEN**

(45) Date de publication du fascicule du brevet: **09.05.84**

(21) Numéro de dépôt: **80401104.7**

(22) Date de dépôt: **24.07.80**

(51) Int. Cl.³: **A 61 B 5/02, A 61 B 10/00**

(54) Appareil d'enregistrement, de contrôle et de dépistage des affections cardio-vasculaires.

(30) Priorité: **24.07.79 FR 7919061**

(43) Date de publication de la demande:
**28.01.81 Bulletin 81/4**

(45) Mention de la délivrance du brevet:
**09.05.84 Bulletin 84/19**

(84) Etats contractants désignés:
**AT BE CH DE GB IT LI LU NL SE**

(56) Documents cités:
**DE - A - 1 466 830**
**FR - A - 2 016 648**
**FR - A - 2 239 974**
**FR - A - 2 299 003**
**US - A - 3 796 213**
**US - A - 3 993 047**
**US - A - 4 063 551**

(73) Titulaire: **Balique, Georges Albert**
**29, rue du Docteur Finlay**
**F-75015 Paris (FR)**

(72) Inventeur: **Balique, Georges Albert**
**29, rue du Docteur Finlay**
**F-75015 Paris (FR)**

(74) Mandataire: **Weinstein, Zinovi et al,**
**Cabinet Z. WEINSTEIN 20, Avenue de Friedland**
**F-75008 Paris (FR)**

Courier Press, Leamington Spa, England.

## Description

L'invention concerne généralement et a essentiellement pour objet un appareil d'enregistrement, de contrôle et de dépistage des affections cardiovasculaires.

De nombreux rapports et statistiques mettent régulièrement l'accent sur l'augmentation sans cesse croissante des maladies cardio-vasculaires qui représentent une des causes importantes de mortalité, ces maladies n'épargnant pas les personnes se considérant d'ellesmêmes, et souvent à tort, comme bien portantes.

La reconnaissance des arythmies cardiaques est essentiellement basée sur l'électrocardiogramme; mais cet examen doit dans tous les cas être pratiqué par un spécialiste dans un cabinet médical. Dans la majorité des cas, cet examen est effectué alors que la maladie est déjà présente; autrement dit, il n'y a pas dépistage précoce de la maladie, alors que les statistiques démontrent qu'un dépistage intial serait hautement bénéfique.

Il est devenu par conséquent indispensable, pour des raisons évidentes, de dépister au maximum ces maladies cardio-vasculaires autrement que par la simple recommandantion d'effectuer périodiquement des contrôles et des examens appropriés. Une telle approche ne peut pas donner, a priori, de résultants satisfaisants, la majorité des personnes étant réticentes à faire des examens dès qu'elles jugent leur état de santé satisfaisant. Un appareil conforme à l'objet du préambule de la revendication 1 est d'autre part connu de la demande de brevet FR—A—2299003.

Le but de l'invention est de contribuer positivement au dépistage et à la prévention des affections cardio-vasculaires grâce à un appareil simple d'emploi et destiné à être mis à la disposition du grand public dans des lieux appropriés. Cet appareil est capable de mettre en évidence immédiatement, sans l'assistance d'un spécialiste, une anomalie du rythme cardiaque. Il n'est pas question, pour un tel appareil, de fournir un électrocardiogramme, mais une information simplifiée suffisamment révélatrice des phénomènes périodiques caractéristiques notamment du rythme cardiaque, et facilement interprétable et exploitable.

L'appareil conforme à l'invention selon la revendication 1 est donc destiné à enregistrer notamment la fréquence et la rythme cardiaque à partir de l'enregistrement du pouls digital recueilli par un procédé pléthysmographique; un tel procédé permet de mettre en évidence le changement de volume d'un ou plusieurs organes sous l'influence des variations de calibre des vaisseaux qui irriguent ce ou ces organes.

Plus précisément, lors de la contraction ventriculaire (systole), le coeur éjecte une certaine quantité de sang qui parcourt les vaisseaux artériels et donne naissance, à un niveau quelconque de système artériel, à un déplacement des parois de l'artère. Ce déplacement est détecté à au moins un endroit sélectionné du corps tel que l'extrémité d'un doigt d'une main par exemple. Ainsi, on obtient une traduction mécanique du passage d'une certaine quantité de sang à un endroit donné du corps.

Cette façon de procéder ne revient pas à enregistrer le pouls, car cela nécessite un appareillage spécial pour obtenir une courbe analogique de déplacement des parois artérielles (sphygmogramme), mais concerne la détection des battements du pouls où les données recueillies sont physiquement élémentaires. Ainsi, avec un tel appareil, on détecte le phénomène mécanique de déplacement des parois d'une artère en un endroit particulier, phénomène qui va se répéter $x$ fois par minute (fréquence par minute), avec un délai entre chaque apparition du phénomène qui est constant ou non (régularité ou irrégularité). Autrement dit, un tel appareil donne un tracé artéfacté constitué par une série d'impulsions identiques, chaque impulsion correspondant à un battement du pouls que l'on détecte par un dispositif de détection approprié.

L'invention propose un appareil conforme à l'appareil revendiqué dans la revendication 1. D'autres caractéristiques de l'invention sont revendiquées dans les revendication 2 à 9.

Parmi les avantages nombreux de l'appareil conforme à l'invention, il faut insister:
— sur sa simplicité d'utilisation,
— sur son rôle de guide de santé, au même titre qu'un thermomètre ou une bascule,
sur le fait qu'il est automatique et n'a nullement besoin ni de l'assistance d'un opérateur pour son fonctionnement, ni d'un spécialiste pour l'interprétation des résultats.

D'autres caractéristiques, avantages et détails apparaîtront plus clairement à l'aide de la description explicative qui va suivre faite en référence aux dessins annexés donnés uniquement à titre d'exemple et dans lesquels:

La figure 1 représente schématiquement plusieurs dispositifs de détection du pouls digital au niveau d'un doigt d'un utilisateur de l'appareil conforme à l'invention.

La figure 2 représente de façon synoptique les principaux circuits de traitement de l'appareil conforme à l'invention.

La figure 3 représente en détail un premier dispositif de détection conforme à l'invention.

La figure 4 représente schématiquement un second dispositif de détection conforme à l'invention.

La figure 5 illustre graphiquement par les tracés *a* et *b* des résultats de tests obtenus par l'appareil conforme à l'invention.

La figure 6 représente schématiquement un circuit corrélateur à prédétermination itérative pour déterminer à l'avance l'arrivée d'une impulsion de test; et

La figure 7 illustre graphiquement le principe de fonctionnement du circuit représenté sur la figure 6.

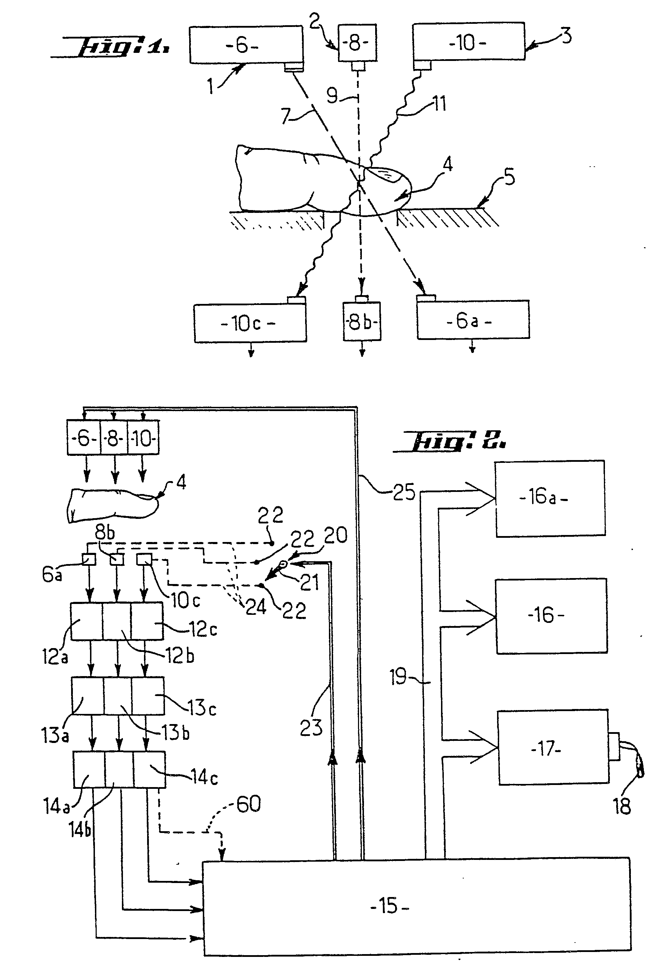

Sur la figure 1 sont représentés schématiquement trois dispositifs de détection 1, 2, 3 de l'appareil conforme à l'invention et destinés chacun à détecter le pouls digital au niveau par exemple d'un doigt 4 de la main d'un utilisateur de l'appareil, doigt 4 qui est apposé sur un appui 5.

Le dispositif de détection 1 est par exemple un dispositif de détection optique classique comprenant un générateur 6 émetteur d'un flux lumineux 7 reçu par un capteur associé 6a.

Le dispositif de détection 2 est par exemple un dispositif de détection à ultra-sons classique de type acoustique ou éventuellement de type électro-magnétique comprenant un générateur 8 émetteur de ces ultra-sons 9 reçu par un capteur associé 8b. A titre d'exemple, la fréquence du faisceau ultra-sonore est de plusieurs mégahertz.

Le dispositif de détection 3 est par exemple un dispositif de détection infrarouge comprenant un générateur 10 d'un rayonnement infrarouge 11 reçu par un capteur associé 10c. A titre d'exemple, le rayonnement infrarouge peut avoir une longueur d'onde de 900 nanomètres.

L'ensemble de ces dispositifs de détection ont été représentés comme travaillant en transmission, c'est-à-dire qu'un générateur et son capteur associé sont respectivement situé de part et d'autre du doigt 4. Il est cependant tout à fait possible de travailler en réflexion profonde, c'est-à-dire qu'un générateur et son capteur associé sont d'un même côté du doigt 4, ce qui est préférable dans le cas par exemple d'un dispositif de détection optique en lumière visible.

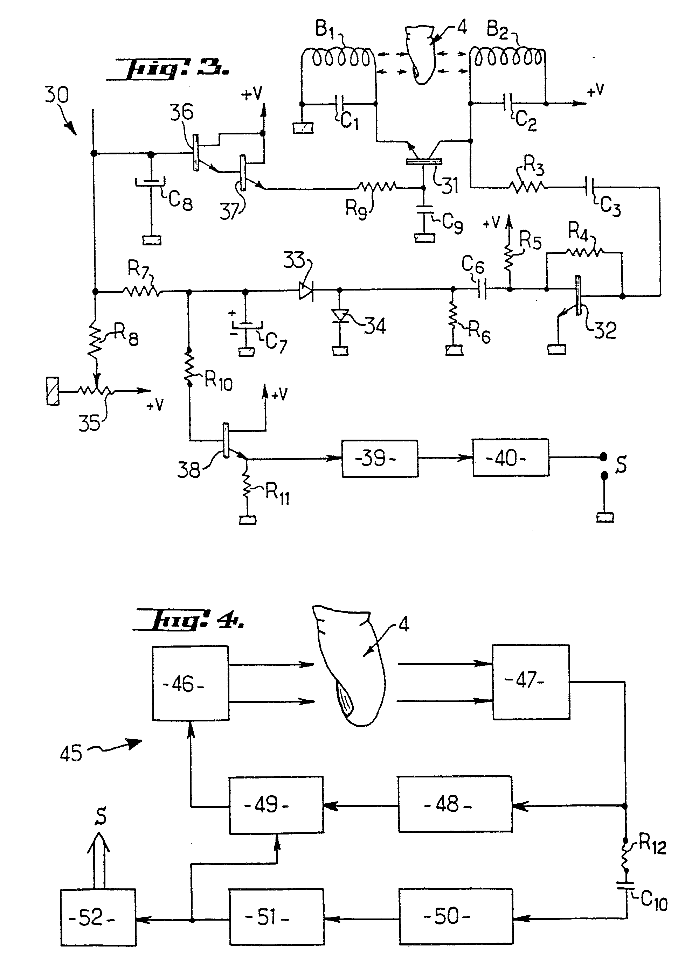

En se reportant à la figure 2, les sorties des capteurs 6a, 8b, 10c sont respectivement reliées aux entrées de trois détecteurs 12a, 12b et 12c. Les sorties de ces trois détecteur 12a, 12b, 12c sont reliées respectivement à trois amplificateurs 13a, 13b et 13c. Les sorties des amplificateurs, après mise en forme (circuits 14a, 14b, 14c) sont reliées aux entrées d'un multiplexeur 15 réalisant plusieurs fonctions permettant notamment:

— la visualisation de la fréquence et du rythme cardiaque de l'utilisateur sur un dispositif d'affichage 16 ou la traduction de la fréquence et du rythme cardiaque par un dispositif sonore 16a.

— l'enregistrement de la fréquence et du rythme cardiaque dans un enregistreur 17 avec impression sur un support et notamment sur une bande de papier filigranée 18 créée pour les besoins; le dispositif de visualisation 16, le dispositif sonore 16a et l'enregistreur 17, sont reliés à des sorties du multiplexeur 15 par l'intermédiaire d'un bus d'information 19, ·

— la sélection éventuelle d'un capteur particulier 6a, 8b ou 10c, en fonction des informations recueillies par le multiplexeur 15 et en provenance de ces capteurs. Pour cela, l'appareil comprend par exemple un sélecteur 20 dont l'armature mobile 21 est commandée automatiquement par un bus d'information 23 venant du multiplexeur 15. Les contact fixes 22 du sélecteur 20 coopèrent avec l'armature mobile 21 dans le but de préférencier l'un ou l'autre des capteurs, et sont reliés respectivement, par des conducteurs 24, aux capteurs 6a, 8b, 10c,

— la modification des fréquences des générateurs 6, 8, 10 éventuellement asservis. Ces générateurs sont reliés au multiplexeur 15 par un bus d'information 25.

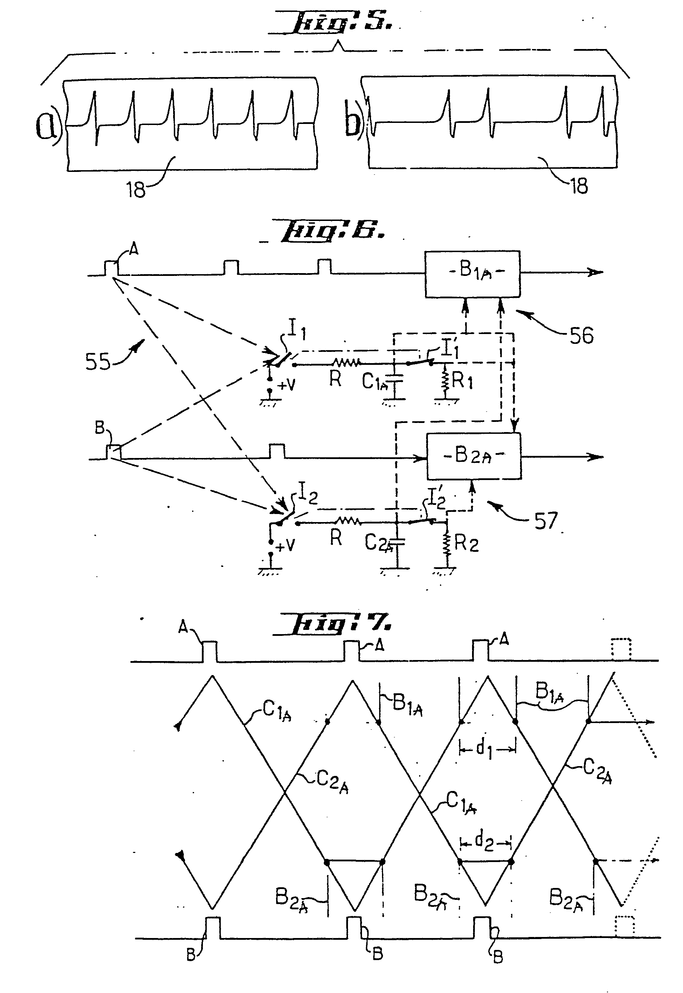

En se référant à la figure 3, il est décrit un dispositif de détection 30 particulier qui est un oscillateur HF à très faible niveau et asservi en boucle d'amplitude.

Ce dispositif de détection 30 comprend un transistor oscillateur 31 à couplage d'émetteur. L'émetteur de ce transistor 31 est relié à un circuit accordé d'émetteur comprenant un bobinage B1 dont une extrémité est reliée à l'émetteur du transistor 31 et dont l'autre extrémité est reliée à la mass. Un condensateur C1 est éventuellement monté en parallèle sur le bobinage B1, pour parfaire l'oscillation.

Le collecteur du transistor oscillateur 31 est relié à un circuit accordé de collecteur comprenant un bobinage B2 dont une extrémité est reliée au collecteur du transistor 31 et dont l'autre extrémité est reliée à une tension d'alimentation (+V). Un conddensateur C2 est monté en parallèle sur le bobinage B2.

Le collecteur de transistor oscillateur 31 est préférentiellement relié à un réseau RC de prélèvement du signal haute fréquence de l'oscillateur et comprend en série une résistance R3 et un condensateur C3.

La borne du condensateur C3 opposée à la résistance R3 est reliée à la base d'un transistor amplificateur 32 dont l'émetteur est relié a la masse. Une résistance R4 de polarisation et de contre-réaction du transistor 32 est montée entre le collecteur et la base de ce transistor. Le collecteur du transistor amplificateur 32 est relié à la source de tension (+V) par l'intermédiaire d'une résistance de charge R5.

Le collecteur du transistor amplificateur 32 est relié à un circuit de découplage différenciateur comprenant un condensateur C6 et une résistance R6 reliée à la masse.

Le circuit différenciateur (R6, C6) est relié à un circuit de détection constitué de deux diodes 33, 34. Le point de liaison entre la résistance R6 et le condensateur C6 est relié à la cathode de la diode 33. L'anode de la diode 34 est reliée à la cathode de la diode 33, alors que sa cathode est reliée à la masse. L'anode de la diode 33 est reliée à un condensateur de filtrage de détection C7.

Le point de liaison entre la diode 33 et le condensateur C7 est relié à deux résistances de limitation R7, R8 montées en série. L'autre extrémité de la résistance R8 est solidaire d'un curseur d'un potentiomètre 35 de réglage de l'amplitude des oscillations du transistor 31, à travers le circuit Darlington ci-après décrit.

Le point de liaison entre les résistance de limitation R7, R8 est relié à un circuit de commande du courant de base du transistor oscillateur 31 avec interposition d'un condensateur de filtrage C8. Ce circuit de commande est constitué par deux transistors 36, 37 montés en circuit "Darlington". L'émetteur du transistor 37 est relié à une résistance R9 de limitation du courant de base du transistor oscillateur 31. Cette résistance est d'une part reliée à la base du transistor 31 et d'autre part à la masse par l'intermédiaire d'un condensateur de découplage haute fréquence C9.

La sortie de ce dispositif de détection 30 s'effectue au niveau point de liaison entre le condensateur C7 (condensateur de détection) et la résistance de limitation R7. Ce point de liaison est relié, par l'intermédiaire d'une résistance R10, à la base d'un transistor amplificateur de courant 38, dont le collecteur est relié à la source d'alimentation (+V) et dont l'émetteur est relié à la masse par l'intermédiaire d'une résistance de charge R11. Le signal amplifié est prélevé sur l'émetteur du transistor amplificateur 38 adaptateur d'impédance, pour attaquer un filtre passebande 39, puis un circuit de mise en forme 40 pour obtenir une information élémentaire exploitable.

En se référant à la figure 4, il ve être décrit un autre type de dispositif de détection conforme à l'invention. Ce dispositif de détection 45 est un dispositif à ultra-sons comprenant un circuit 46 émetteur d'ultra-sons et un circuit 47 récepteur des ultra-sons émis. A cet émetteur 46 et à ce récepteur 47 sont associés une boucle d'oscillation et une boucle de couplage. La boucle d'oscillation comprend un amplificateur 48 dont l'entrée est reliée à la sortie du récepteur 47. La sortie de l'amplificateur 48 est reliée à l'entrée d'un atténuateur 49 dont la sortie est reliée à l'émetteur d'ultra-sons 46.

La boucle de couplage comprend un circuit de prélèvement des oscillations constitué par une résistance R12 et un condensateur C10 montés en série, ou éventuellement par un tout autre système de couplage. La résistance R12 est reliée à la sortie du récepteur d'ultra-sons 47, et le condensateur C10 est relié à un amplificateur de mesure 50. La sortie de cet amplificateur est reliée à un étage de détection 51 qui effectue une conversion alternatifcontinu des signaux qu'il reçoit. Les signaus continus en sortie de l'étage détecteur 51 permettent de commander l'atténuateur 49 qui est en atténuateur à commande par courant continu exemple. Les signaux de rattrapage d'asservissement de boucle en sortie du détecteur 51 sont également traités et mis en forme par des circuits 52 afin d'obtenir en sortie S un signal exploitable par le multiplexeur 15.

L'appareil comprend également au moins un circuit corrélateur à prédétermination itérative qui est représenté schématiquement sur la figure 6 et qui permet de prédéterminer l'arrivée d'une impulsion issue d'un dispositif de détection en fonction d'au moins une des impulsions précédentes déjà détectées au niveau d'un autre dispositif de détection. Ce circuit 55 comprend:

— un premier circuit 56 comprenant essentiellement un circuit de charge (tension v, résistance R) à courant constant d'un condensateur C1A avec interposition d'un interrupteur I1 et un circuit de décharge également à courant constant (résistance R1 avec interposition d'un interrupteur I'1, par exemple mécaniquement accouplé à l'interrupteur I1) du condensateur C1A. Le circuit de décharge est relié aux entrées de commande de deux bascules à seuil B1A et B2A.

— un second circuit 57 identique au précédent et comprenant un circuit de charge (tension v, résistance R) à courant constant d'un condensateur C2A avec interposition d'un interrupteur I2, et un circuit de décharge également à courant constant du condensateur C2A constitué par une résistance R2 avec interposition d'un interrupteur I'2, mécaniquement accouplé à l'interrupteur I2 par exemple. Ce circuit de décharge est relié aux entrées de commande des deux bascules à seuil B1A et B2A.

Les liaisons de ce circuit corrélateur à prédétermination itérative 55 seront décrites plus en détail par la suite lors de l'explication du fonctionnement de l'appareil.

Le fonctionnement de l'appareil précédemment décrit est le suivant.

L'utilisateur désireux d'effectuer un test commence tout d'abord par mettre l'appareil en fonctionnement par tout moyen approprié comme par exemple: présence du doigt, bouton-poussoir, introduction d'une pièce de monnaie dans une fente de l'appareil.

Dans le cas où l'appareil est équipé des dispositifs de détection classiques 1, 2, 3 (figures 1 et 2), la mise sous tension de l'appareil provoque l'excitation des générateurs 6, 8, 10. En l'absence d'un corps étranger interceptant les flux 7, 9, 11 émis respectivement par les générateurs 6, 8, 10, les capteurs 6a, 8b, 10c, associés respectivement aux générateurs reçoivent l'intégralité des flux émis. Les détecteurs 12a, 12b, 12c associés respectivement aux capteurs 6a, 8b, 10c sensibles à des variations d'amplitude des signaux électriques fournis respectivement par les capteurs 6a, 8b, 10c, ne délivrent en fait aucun signal en sortie puisqu'il n'y a pas de perturbation au niveau des flux 7, 9, 11 émis respectivement par les générateurs 6, 8, 10. Le multiplexeur 15 ne reçoit aucun signal et il peut être prévu au niveau de dispositif d'affichage 16, une lampe témoin signifiant uniquement que l'appareil est sous tension.

Après cette mise sous tension, l'ultilisateur vient positionner correctement un doigt de sa main à un endroit clairement indiqué sur l'appareil, de façon à venir perturber les différents flux émis par l'ensemble des générateurs des dispositifs de détection 1, 2, 3.

Le flux 7 du générateur 6 va être en partie absorbé par le doigt 4 de l'utilisateur, si bien que le capteur 6a associé au générateur 6 délivrera un signal de moindre amplitude. Cette variation d'amplitude va être détectée par le détecteur 12a qui, à son tour, va produire un signal amplifié par l'amplificateur 13a associé et mis en forme par le circuit de mise en forme 14a associé. Ce signal est ensuite reçu par le multiplexeur 15.

Les flux 9 et 11 des générateurs 8 et 10 vont également être perturbés, et les signaux émis par les capteurs 8 et 10 vont être traités d'une façon identique au signal émis par le capteur 6.

Dans l'exemple illustré, le multiplexeur 15 va donc recevoir trois signaux qui, en fait, ne sont pas intéressants pour le test, car ils indiquent seulement la présence d'un corps étranger dans le chemin des flux des dispositifs de détection 1, 2, 3. Si ce corps étranger est inerte, le multiplexeur ne recevra plus aucun signal. Par contre, si le corps étranger a un volume qui varie, l'énergie absorbée par le corps va varier, ce qui va se traduire par des variations d'amplitude et/ou de fréquence dans les signaux en sortie des capteurs 6, 8, 10. Dans l'application envisagée, ce changement de volume va se situer au niveau des vaisseaux qui vont se dilater à chaque afflux de sang. Autrement dit, il y aura des variations d'amplitude dans les signaux émis par les capteurs 6, 8, 10. Ces variations d'amplitude vont par conséquent se traduire par des signaux qui vont être reçus par le multiplexeur 15.

Le rôle du multiplexeur est de comparer, de mélanger, de corréler, d'intrpolar, etc ...., ces différents signaux reçu afin de les interpréter pour en extraire des informations exhaustives à haute fiabilité et à très forte probabilité de véracité d'une part, ainsi que des informations secondaires indirectes ou indirectement déduites d'autre part.

Ces informations et notamment la fréquence du pouls digital sont ensuite envoyées, par l'intermédiaire du bus d'information 19, vers le dispositif de visualisation 16, et/ou vers le dispositif sonore 16a, et/ou vers le dispositif d'enregistrement 17 avec impression sur la bande de papier filigranée 18.

Sur la figure 5 sont représentés respectivement deux tracés artéfactés a, b qui représentent respectivement deux fréquences cardiaques imprimées sur la band filigranée 18. Le tracé artéfacté a montre une fréquence cardiaque normale, alors que le tracé artéfacté b montre une fréquence cardiaque anormale. L'interprétation de ces tracés artéfactes est évidente pour l'utilisateur qui pourra en tirer les conclusions qui s'imposent.

Par l'intermédiaire du bus d'information 25 qui sort du multiplexeur, il est possible de modifier les caractéristiques des générateurs de flux 6, 8, 10, s'il s'avère par exemple que les signaus reçus par le multiplexeur 15 présentent des anomalies de fonctionnement. De la même façon, il est possible de jouer sur le sélecteur 20 pour sélectionner par exemple un capteur particulier qui est susceptible de donner les meilleurs résultats. En effet, les dispositifs de détection étant de nature physique différente, ils peuvent réagir différemment en fonction de l'utilisateur.

En se référant à la figure 3, il va être décrit le fonctionnement du dispositif de détection 30 qui peut avantageusement remplacer l'un des dispositifs de détection 1, 2, 3 de la figure 1.

Une fois dispositif de détection 30 mis sous tension, le transistor oscillateur 31 produit des oscillations à très faible niveau, l'ordre de la centaine de millivolts. Si l'on introduit un corps étranger entre les deux bobinages B1 et B2 du circuit d'émetteur et du circuit accordé collecteur respectivement, il va y avoir une chute de l'amplitude des oscillations tant par modification du coefficient de surtension du circuit accordé que par modification du couplage entre l'émetteur et le collecteur du transistor oscillateur 31. Comme précédemment, les afflux de sang dans les vaisseaux du doigt 4 interposé entre les deux bobinages B1 et B2, vont modifier à chaque fois les amplitudes des oscillations du transistor 31.

Par l'intermédiaire de la résistance R3 et du condensateur C3 montés en série, on prélève une très faible partie des oscillations présentes au collecteur du transistor 31. Il est indispensable de ne prélever qu'un très faible signal pour éviter d'introduire un trop grand affaiblissement qui ferait écrouler les oscillations. Pour cette raison, le couplage est très faible, et dans le cas décrit, la résistance R3 est relativement élevée.

Le signal prélevé par le circuit de prélèvement (R3, C3) est amplifié ensuite par le transistor amplificateur 32. Le signal amplifié est ensuite découplé par l'intermédiare du circuit différenciateur constitué par le condensateur C6 et la résistance R6. Puis, par l'intermédiaire de la diode 34 on exclut les impulsions positives pour ne conserver que les impulsions négatives qui passent par la diode 33. Ce signal négatif est ensuite retranché du signal initial de réglage du transistor oscillateur 31 donné par la résistance R8 et le potentiomètre 35. La tension au point de liaison de la résistance R7 et R8 diminue, c'est-à-dire que l'on abaisse la tension de base du transistor 36 et par conséquent la tension de base du transistor oscillateur 31. Dans ces conditions, on augmente la tension collecteur-base de ce transistor, ce qui permet de rattraper la chute des oscillations provoquée par les afflux de sang dans le doigt 4. On a par conséquent un oscillateur très haute fréquence à très faible niveau asservi en boucle d'amplitude. Les impulsions négatives en sortie de la diode 33 en provenance d'un rattrapage électronique de boucle d'asservissement sont prélevées et amplifiées par le transistor amplificateur 38 et traitées par les circuits de traitement 39 et 40 pour obtenir à la sortie S des impulsions exploitables par le multiplexeur 15.

En se référant à la figure 4, il va être décrit un autre mode de réalisation d'un dispositif de détection 45 conforme à l'invention.

Ce dispositif de détection 45 est un dispositif à ultra-sons ultilisant l'effet Larsen. Les ultra-sons émis par l'émetteur 46 sont reçus par le récepteur 47. Les impulsions d'oscillation en sortie du récepteur 47 sont amplifiées par l'amplificateur 48 puis réinjectées dans l'émetteur 46 avec interposition de l'atténuateur commandable 49. Dans ces conditions, on réinjecte une partie de l'énergie de sortie à l'entrée pour entretenir le oscillations. Comme précédemment, l'interposition du doigt 4 de l'utilisateur entre l'émetteur 46 et le récepteur 47 va provoquer, ainsi que les afflux de sang dans le doigt 4, des variations d'amplitude des oscillations au niveau du récepteur 47. On prélève, par l'intermédiaire de la résistance R12 et du condensateur C10, une très faible partie du signal issu de générateur 47. Ce faible signal est amplifié par l'amplificateur 50. Comme dans les cas du dispositf de détection décrit sur la figure 3, il ne faut prélever qu'un faible signal pour ne pas perturber les oscillations. Le signal alternatif prélevé est ensuite converti par le détecteur 51 en un signal continu qui permet de commander l'atténuateur 49 afin de rattraper les chutes d'osicllations provoquées par les afflux de sang dans le doigt 4 de l'utilisateur. Le signal en sortie du détecteur 51 est traité par les circuits de mise en forme 52, d'une façon classique, pour donner à la sortie S des impulsions exploitables par le multiplexeur 15.

En se référant aux figures 6 et 7, il va être décrit le fonctionnement du circuit corrélateur à prédétermination itérative de manière à prédéterminer l'arrivée d'une impulsion issue d'un dispositif de détection en fonction d'au moins une des impulsions précédemment détectées par un autre dispositif de détection.

Supposons qu'une impulsion A se présente à l'entrée du circuit corrélateur 55. Cette impulsion A va ouvrir l'interrupteur I2 du circuit 57 et fermer l'interrupteur I'2 de façon à permettre la décharge à courant constant du condensateur C2A (précédemment chargé) dans la résistance R2. Lorsque la tension aux bornes du condensateur C2A atteint la tension de seuil de la bascule B2A, celle-ci est rendue passante. A partie de cet instant, la bascule B2A laisse passer les impulsions qu'elle reçoit à son entrée et provenant d'un second dispositif de détection. Normalement, quelques instants plus tard, une impulsion B issue du second dispositif de détection doit apparaître et cette impulsion va provoquer le basculement de l'interrupteur I2 du circuit 57 pour permettre à nouveau la charge à courant constant du condensateur C2A, lequel ne peut plus par ailleurs se décharger dans la résistance R2 car l'interrupteur I'2 est ouvert en synchronisme avec l'interrupteur 12. Lorsque la charge de condensateur C2A atteint à nouveau le seuil de basculement de la bascule B2A, celle-ci devient non passante. On a ainsi déterminé une durée *d1* pendant laquelle la bascule B2A est passante.

Le condensateur C2A continuant à se charger va atteindre le seuil de basculement de la bascule B1A et rendre celle-ci passante en prévision de l'apparition d'une impulsion A. A l'arrivée de cette impulsion A du premier détecteur, l'interrupteur I2 est à nouveau commuté pour assurer la décharge à courant constant du condensateur C2A. En se déchargeant, la tension du condensateur C2A repasse par le seuil de déclenchement de la bascule B1A pour refermer celle-ci. On a donc déterminé une durée *d2* pendant laquelle la bascule B1A est rendue passante pour laisser passer les informations provenant du premier dispositif de détection.

Si les prévisions sont exactes, les bascules B1A et B2A seront rendues passantes juste avant l'apparition d'une impulsion, puis refermées.

Par contre, si l'impulsion B n'arrive pas une fois la bascule B2A rendue passante, celle-ci restera passante et laissera passer toutes les informations provenant du deuxième dispositif de détection. Autrement dit, l'information finale ne sera pas très sélective.

Le raisonnement qui vient d'être fait en considérant comme point de départ l'arrivée d'une impulsion A, est exactement le même en considérant l'arrivée d'une impulsion B, en vue de déterminer l'apparition de la prochaine impulsion A. Dans ce cas, c'est le circuit 56 qui est à considérer. Dans le réalité, les deux circuits 56 et 57 marchent quasiment simultanément.

Il est possible dans le cas de la non apparition d'une impulsion A ou B de compenser cette absence par l'introduction d'une impulsion qui permet de ramener le condensateur C1A ou C2A dans les limites de fonctionnement normales. Ce circuit 55 permet donc une bonne sélectivité de l'information notamment lorsque le rythme cardiaque est relativement régulier.

Ce circuit corrélateur peut être placé en amont du multiplexeur 15, c'est-à-dire que les impulsions qui passent par les bascules B1A et B2A sont transmises au multiplexeur 15 en vue d'assurer l'affichage et/ou l'audition et/ou l'enregistrement du rythme cardiaque.

Dans l'exemple décrit, on a considéré que les trois dispositifs de détection étaient associés à un seul doigt 4 de l'utilisateur, mais bien évidemment on peut prévoir ces dispositifs sur plusieurs doigts de la main. Le dispositif de détection représenté sur la figure 3 peut bien évidemment être réalisé avec des circuits intégrés.

Il est à noter également que le multiplexeur 15 peut recevoir des informations analogiques à des fins d'asservissement éventuels, à partir des circuits de traitement des signaux venant des capteurs, cette possibilité a été matérialisée sur la figure 2 par la liaison en pointillés 60.

L'appareil qui vient d'être décrit peut inclure également d'autres circuits notamment associés au multiplexeur 15 pour permettre d'indiquer, par exemple au niveau du dispositif de visualisation 16, des indications relatives à la véracité des informations qui seront transcrites par exemple sur la bande de papier filigranée 18.

Les enregistrements obtenus par l'appareil sont élémentaires mais rigoureux, c'est-à-dire qu'il permettent le contrôle du rythme et de la fréquence cardiaque. Un rythme anormal pourra être facilement dépisté après avoir effectué éventuellement un second test pour confirmer le premier.

Il est également possible en utilisant plusieurs dispositifs de détection de mesurer la propagation du flux sanguin dans les artères. Avec un premier dispositif de détection tel qu'un capteur par palpation placé sur le coeur, on détecte un battement de ce dernier, et avec un second dispositif de détection tel que ceux décrits précédemment, on détecte le moment où se produit l'afflux sanguin par exemple au niveau d'un doigt de l'utilisateur. A partir de ces deux mesures, il est facile de connaître la vitesse de progagation.

Un tel appareil peut par conséquent être mis facilement à la disposition du grand public et aider considérablement le contrôle et le dépistage des affections cardio-vasculaires.

Bien entendu, l'invention n'est nullement limitée aux modes de réalisation décrits et représentés qui n'ont été donnés qu'à titre d'exemple; elle comprend tous les moyens constituant des équivalents techniques des moyens décrits, ainsi que leurs combinaisons, si celles-ci sont exécutées suivant son esprit et mises en oeuvre dans le cadre des revendications qui suivent.

**Revendications**

1. Appareil d'enregistement, de contrôle et de dépistage d'affections cardio-vasculaires par la détection, l'enregistrement et/ou l'audition et/ou la visualisation de la fréquence et du rythme cardiaque à l'aide de l'enregistrement du pouls digital recueilli par un procédé pléthysmographique à un doigt (4) d'une main, comprenant un dispositif de détection (1,2,3,30,45) du pouls digital constitué par un générateur de flux déterminé (6,8,10,B1,B2,46) et un capteur associé (6a, 8b, 10c, B1, B2, 47), des circuits traitement (12a, 12b, 12c, 13a, 13b, 13c, 14a, 14b, 14c, 15), un dispositif enregistreur (17) et/ou un dispositif sonore (16a) et/ou un dispositif de visualisation (16) des signaux issus du dispositif de visualisation (16) des signaux issus du dispositif de détection, caractérisé en ce qu'il comprend au moins deux dispositifs de détection (1,2,3,30,45) de nature physique différente disposés autour du doigt de la main et détectant périodiquement le même pouls digital, les signaux de sortie des dispositifs de détection étant traités séparément et/ou ensemble dans une unité de multiplexage (15) reliée par l'intermédiaire d'un bus d'information (19) aux dispositifs (16,16a,17) pour assurer l'affichage ou l'enregistrement ou l'audition desdits signaux représentatifs de la fréquence et du rythme cardiaque, ladite unité de multiplexage commandant par l'intermédiaire d'un autre bus d'information (23) un organe sélecteur (20) de l'un des dispositifs de détection en fonction des signaux de sortie des dispositifs de détection et susceptible de donner la meilleure information relative à la fréquence et au rythme cardiaque.

2. Appareil selon la revendication 1, caractérisé en ce que l'un des dispositifs de détection (30) précités comprend un oscillateur haute fréquence à très bas niveau, avec présence d'une boucle d'asservissement d'amplitude et constitué par un transistor oscillateur (31) comprenant un circuit accordé (B1,C1) dans son circuit d'émetteur et un circuit accordé (B2,C2) dans son circuit collecteur, un circuit de prélèvement (R3,C3), au niveau du collecteur du transistor, d'une faible partie du signal haute fréquence dudit transistor (31), un amplificateur (32) dudit signal prélevé, un réseau différenciateur (R6,C6) pour découpler ledit signal amplifié, un circuit détecteur (33,34) pour détecter le signal différencié, ce signal détecté étant reçu par un circuit de commande (36,37) du courant de base du transistor oscillateur.

3. Appareil selon la revendication 2, caractérisé en ce que le circuit de command du courant de base précité est constitué par deux transistors (36,37) montés suivant un montage Darlington.

4. Appareil selon la revendication 2, caractérisé en ce qu'un signal est prélevé à la sortie du circuit détecteur précité, signal qui est envoyé dans des circuits de mise en forme (40) puis au multiplexeur (15) pour assurer son affichage sur le dispositif de visualisation (16) précité ou son enregistrement sur une bande de papier (18) à partir du dispositif d'enregistrement (17) précité ou son audition à partir du dispositif sonore (16a) précité.

5. Appareil selon la revendication 1, caractérisé en ce que l'un des dispositifs de détection précités est un dispositif à ultra-sons (45) comprenant un circuit émetteur (46) d'ultra-sons, un circuit récepteur (47) des ultra-sons émis, une boucle d'oscillation (48,49) comprenant un amplificateur (48) dont l'entrée est reliée à la sortie de récepteur ultra-sons précité, et en ce que la sortie de l'amplificateur (48) est reliée à l'entrée d'un atténuateur dynamique (49) dont la sortie est reliée à l'émetteur d'ultra-sons (46) précité, la boucle de couplage précitée comprenant un circuit de prélèvement (R12,C10) des oscillations reçues par le récepteur (47) d'ultra-ons précité et constitué par une résistance (R12) et un condensateur (C10) montés en série, la résistance (R12) étant reliée à la sortie dudit récepteur (47) d'ultra-sons alors que le condensateur (C10) est relié à un amplificateur de mesure (50), et en ce que la sortie dudit amplificateur (50) est reliée à un étage de détection (52) effectuant une conversion alternatif-continu des signaux qu'il reçoit, les signaux en sortie dudit étage détecteur (51) assurant la commande de l'atténuateur (49) précité.

6. Appareil selon la revendication 5, caractérisé en ce que des signaux sont prélevés en sortie du détecteur (51) précité, puis envoyés à des circuits de mise en forme (52) pour être traités par le multiplexeur (15) précité en vue de leur affichage sur le dispositif de visualisation précité (16), ou leur enregistrement (17) sur une bande (18) par exemple à partir du dispositif d'enregistrement (17) précité ou leur audition à partir du dispositif sonore (16a) précité.

7. Appareil selon l'une des revendications précédentes, caractérisé en ce qu'il comprend également au moins un circuit (55) de prédétermination de l'arrivée d'une impulsion issue d'un des dispositifs de détection précités en fonction d'au moins une des impulsions précédentes déjà détectées par un autre dispositif de détection (1,2,3,30,45) précité.

8. Appareil selon la revendication 7, caractérisé en ce que le circuit (55) précité comprend au moins deux condensateurs (C1A,C2A) dont la charge et la décharge sont sensiblement constantes, et au moins deux bascules (B1A,B2A) à seuil associées auxdits condensateurs (C1A,C2A).

9. Appareil selon la revendication 8, caractérisé en ce que les deux bascules (B1A,B2A) précitées sont associées à deux dispositifs de détection (1,2,3,30,45) précités pour laisser passer ou non des impulsions émises par ces dispositifs de détection pendant un temps prédétermine par les condensateurs (C1A,C2A) précités.

**Claims**

1. Apparatus for recording, controlling and early detecting cardiovascular diseases through the detection, the recording and/or the hearing and/or the visualizing of the cardiac frequency and rhythm by means of the recording of the digital pulse picked up through a plethysmographic process on one finger (4) of one hand, comprising a digital pulse detecting device (1,2,3,30,45) consisting of a determined flux generator (6,8,10,B1,B2,46) and an associated sensor (6a,8b,10c,B1,B2,47), processing circuits (12a, 12b, 12c, 13a, 13b, 13c, 14a, 14b, 14c, 15), issuing from the detection device, characterized in that it comprises at least two detecting devices (1,2,3,30,45) of different physical nature arranged about the finger of the hand and periodically detecting the same digital pulse, the output signals from the detecting devices being processed separately and/or together in a multiplexing unit (15) connected through the agency of a data bus (19) to the devices (16,16a,17) for providing the display or recording or hearing of the said signals representative of the cardiac frequency and rhythm, the said multiplexing unit operating through the medium of another data bus (23) a selector member (20) of one of the detecting devices in accordance with output signals from the detecting devices and likely to give the best information relative to the cardiac frequency and rhythm.

2. Apparatus according to claim 1, characterized in that one of the aforesaid detecting devices (30) comprises a high frequency oscillator with a very low level with the provision of an amplitude feedback loop and consisting of an oscillating transistor (31) comprising a tuned circuit (B1,C1) in its emitter circuit and a tuned circuit (B2,C2) in its collector circuit, a circuit (R3,C3) for picking up at the collector of the transistor a small part of the high frequency signal from the said transistor (31), an amplifier (32) for the said picked up signal, a differentiating network (R6,C6) for decoupling the said amplified signal, a detecting circuit (33,34) for detecting the differentiated signal, this detected signal being received by a circuit (36,37) for controlling the base current of the oscillating transistor.

3. Apparatus according to claim 2, characterized in that the aforesaid circuit for controlling the base current consists of two transistors (36,37) arranged according to a Darlington connection.

4. Apparatus according to claim 2, characterized in that a signal is picked up at the output of the aforesaid detecting circuit, which signal is fed into shaping circuits (40) and then to the multiplexer (15) to provide its display onto the aforesaid visualizing device (16) or its recording on a paper tape (18) from the aforesaid recording device (17) or its hearing from the aforesaid sound device (16a).

5. Apparatus according to claim 1, characterized in that one of the aforesaid detecting devices is an ultra-sound device (45) comprising an ultra-sound emitting circuit (46), a circuit (47) for receiving the emitted ultrasounds, an oscillation loop (48,49) comprising an amplifier (48) the input of which is connected to the output of the aforesaid ultra-sound receiver and in that the output of the amplifier (48) is connected to the input of a dynamic attenuator (49) the output of which is connected to the aforesaid ultra-sound emitter (46), the aforesaid coupling loops comprising a circuit (R12,C10) for picking up the oscillations received by the aforesaid ultra-sound receiver (47) and consisting of a resistor (R12) and a capacitor (C10) connected in series, the resistor (R12) being connected to the output of the aforesaid ultra-sound receiver (47) whereas the capacitor (C10) is connected to a measuring amplifier (50), and in that the output of the said amplifier (50) is connected to a detection stage (52) effecting an a.c. — d.c. conversion of the signals it receives, the output signals of the said detecting range (51) providing for the control of the aforesaid attenuator (49).

6. Apparatus according to claim 5, characterized in that signals are picked up at the output of the aforesaid detector (51) and then fed to shaping circuits (52) for being processed by the aforesaid multiplexer (15) for being processed by the aforesaid multiplexer (15) with a view to their being displayed onto the aforesaid visualizing device (16) or their being recorded (17) onto a tape (18) for instance from the aforesaid recording device (17) or their being heard from the aforesaid sounding device (16a).

7. Apparatus according to one of the foregoing claims, characterized in that it also comprises at least one circuit (55) for predetermining the arrival of one impulse issuing from one of the aforesaid detecting devices in accordance with at least one of the foregoing impulses already detected by another aforesaid detection device (1,2,3,30,45).

8. Apparatus according to claim 7, characterized in that the aforesaid circuit (55) comprises at least two capacitors (C1A,C2A) the charge and the discharge of which are substantially constant, and at least two threshold flip-flop circuits (B1A,B2A) associated with the said capacitors (C1A,C2A).

9. Apparatus according to claim 8, characterized in that both aforesaid flip-flop circuits (B1A,B2A) are associated with two aforesaid detecting devices (1,2,3,30,45) for letting through or not impulses emitted by these detection devices for a time predetermined by the aforesaid capacitors (C1A,C2A).

**Patentansprüche**

1. Gerät zur Aufnahme, zur Kontrolle und zum Aufspüren von kardiovaskulären Krankheiten durch die Ermittlung, die Aufnahme und/oder das Anhören und/oder die Sichtbarmachung der Herzfrequenz und des Herzrhythmus mit Hilfe der Aufnahme des über ein plethysmografisches Verfahren an einem Finger (4) einer Hand aufgenommen Fingerpulses, mit einer aus einem einen bestimmten Fluss erzeugenden Generator (6,8,10,B1,B2,46) und einem zugeordneten Messfühler (6a, 8b, 10c, B1, B2, 47) bestehenden Fingerpulsermittlungsvorrichtung (1, 2, 3, 30, 45), Verarbeitungsschaltungen (12a, 12b, 12c, 13a, 13b, 13c, 14a, 14b, 14c, 15) einer Aufnahmevorrichtung (17) und/oder eine schallende Vorrichtung (16a) und/oder einer Vorrichtung (16) zum Sichtbarmachen der von der Ermittlungsvorrichtung herrührenden Signale, dadurch gekennzeichnet, dass es wenigstens zwei um den Finger der Hand herum angeordnete und periodisch den selben Fingerpuls nachweisende Ermittlungsvorrichtungen (1,2,3,30,45) von unterschiedlicher physischer Beschaffenheit aufweist, wobei die Ausgangssignale der Ermittlungsvorrichtungen getrennt und bzw. oder zusammen in einer Multiplexeinheit (15) verarbeitet werden, welche über einen Datenbus (19) an die Vorrichtungen (16,16a,17) zur Anzeige bzw. zur Aufnahme bzw. zum Anhören der besagten die Herzfrequenz un den Herzrhythmus darstellenden Signale angeschlossen ist, wobei die besagte Multiplexeinheit über einen anderen Datenbus (23) ein Wählerglied (20) einer der Ermittlungsvorrichtungen in Abhängigkeit der Ausgangssignale der Ermittlungsvorrichtungen steuert und die beste Nachricht bezüglich der Herzfrequenz und des Herzrhythmus geben kann.

2. Gerät nach Anspruch 1, dadurch gekennzeichnet, dass eine der vorgenannten Ermittlungsvorrichtungen (30) einen Hochfrequenzschwingerzeuger mit sehr niedrigem Pegel bei Vorhandensein einer Amplitudenrückkopplungsschleife aufweist und durch einen Schwingtransistor (31) gebildet wird, welcher einen abgestimmten Kreis (B1,C1) in seiner Emitterschaltung und einer abgestimmten Kreis (B2,C2) in seiner Kollektorschaltung aufweist, sowie eine Schaltung (R3,C3) zur Entnahme am Kollektor des Transistors eines kleinen Teiles des Hochfrequenzsignals des besagten Transistors (31), einen Verstärker (32) des besagten entnommenen Signals, eine Differenzierschaltung (R6,C6) zum Entkoppeln des besagten verstärkten Signals, eine Suchschaltung (33,34) zum Ermitteln des differenzierten Signals, wobei dieses ermittelte Signal durch eine Schaltung (36,37) zur Steuerung des Basisstromes des Schwingtransistors empfangen wird.

3. Gerät nach Anspruch 2, dadurch gekennzeichnet, dass die vorgenannte Basisstromsteuerschaltung aus zwei gemäss einer Darlington-Schaltung angeordneten Transistoren (36,37) besteht.

4. Gerät nach Anspruch 2, dadurch gekennzeichnet, dass ein Signal am Ausgang der vorgenannten Suchschaltung entnommen wird, welches Signal in Impulsformerschaltungen (40) und dann in die Multiplexschaltung (15) gesandt wird, um seine Anzeige auf der vorgenannten Sichtbarmachungsvorrichtung (16) oder seine Aufnahme auf einem Papierstreifen (18) aus der vorgenannten Aufnahmevorrichtung (17) oder sein Anhören an der vorgenannten schallenden Vorrichtung (16a) zu gewährleisten.

5. Gerät nach Anspruch 1, dadurch gekennzeichnet, dass eine der vorgenannten Ermittlungsvorrichtungen eine Ultra-schallvorrichtung (45) ist, welche eine Ultraschallsenderschaltung (46), eine Schaltung (47) zum Empfang der ausgesandten Ultraschalle, eine Schwingschleife (48,49) mit einem Verstärker (48), dessen Eingang an den Ausgang des vorgenannten Ultraschallempfängers angeschlossen ist, aufweist und dass der Ausgang des Verstärkers (48) an den Eingang eines dynamischen Abschwächers (49), dessen Ausgang mit dem vorgenannten Ultraschallsender (46) verbunden ist, angeschlossen ist, wobei die vorgenannte Kopplungsschleife eine Schaltung (R12,C10) zur Entnahme der durch den vorgenannten Ultraschallempfänger (47) empfangenen Schwingungen umfasst, welche Schaltung durch einen Widerstand (R12) und einen Kondensator (C10), die in Reihe geschaltet sind, gebildet wird, wobei der Widerstand (R12) an den Ausgang des bestagen Ultraschallempfängers (47) angeschlossen ist, während der Kondensator (C10) an einen Messverstärker (50) angeschlossen ist, und dass der Ausgang des besagten Verstärkers (50) an eine Detektorstufe (52) angeschlossen ist, welche eine Wechselstrom- Gleichstromumwandlung der von ihr empfangenen Signale durchführt, wobei die Ausgangssignale der besagten Detektorstufe (51) die Steuerung des vorgenannten Abschwächers (49) bewerkstelligen.

6. Gerät nach Anspruch 5, dadurch gekennzeichnet, dass Signale am Ausgang des vorgenannten Detektors (51) entnommen und dann zu Impulsformerschaltungen (52) gesandt werden, um durch der vorgenannten Multiplex (15) verarbeitet zu werden, zum Zweck ihrer Anzeige auf der vorgenannten Sichtbarmachungsvorrichtung (16) oder deren Aufnahme (17) auf einem Streifen (18) z.B. aus der vorgenannten Aufnahmevorrichtung (17) oder deren Anhörung an der vorgenannten schallenden Vorrichtung (16a).

7. Gerät nach einem der vorangehenden Ansprüche, dadurch gekennzeichnet, dass es auch wenigstens eine Schaltung (55) zur Vorbestimmung des Ankommens eines aus einer der vorgenannten Ermittlungsvorrichtungen austretenden Impulses in Abhängigkeit von wenigstens einem der vorangehenden, von einer anderen vorgenannten Ermittlungsvorrichtung (1,2,3,30,45) bereits nachgewiesenen Impulse, umfasst.

8. Gerät nach Anspruch 7, dadurch gekennzeichnet, dass die vorgenannte Schaltung (55) wenigstens zwei Kondensatore (C1A,C2A) deren Ladung und Entladung im wesentlichen konstant sind und wenigstens zwei den besagten Kondensatoren (C1A,C2A) zugeordnete Schwellwertkippschaltungen (B1A,B2A) umfasst.

9. Gerät nach Anspruch 8, dadurch gekennzeichnet, dass die beiden vorgenannten Kippschaltungen (B1A,B2A) zwei vorgenannten Ermittlungsvorrichtungen (1,2,3,30,45) zugeordnet sind, um durch diese Ermittlungsvorrichtungen während einer durch die vorgenannten Kondensatoren (C1A,C2A) vorbestimmten Zeit ausgesandten Impulse durchzulassen oder nicht durchzulassen.

Fig. 1.

Fig. 2.

Fig. 3.

Fig. 4.

**_Fig: 5._**

a)    b)

18    18

**_Fig: 6._**

A    $-B_{1A}-$

55    56

$I_1$  R  $C_{1A}$  $I_1'$  $R_1$
+V

B    $-B_{2A}-$

57

$I_2$  R  $C_{2A}$  $I_2'$  $R_2$
+V

**_Fig: 7._**

A    A

$C_{1A}$  $B_{1A}$  $B_{1A}$

$d_1$

$C_{2A}$  $C_{2A}$

$C_{1A}$

$d_2$

$B_{2A}$  $B_{2A}$  $B_{2A}$

B    B    B